Europäisches Patentamt

European Patent Office

Office européen des brevets

(19)

(11) Publication number: **0 049 461**
**B1**

# (12) EUROPEAN PATENT SPECIFICATION

(45) Date of publication of patent specification: **02.04.86**

(51) Int. Cl.⁴: **A 61 M 1/32**

(21) Application number: **81107732.0**

(22) Date of filing: **29.09.81**

(54) **Device for oxygenating blood in extrabodily circulation with heat exchanger.**

(30) Priority: **06.10.80 IT 2514180**
**10.07.81 IT 2286481**

(43) Date of publication of application:
**14.04.82 Bulletin 82/15**

(45) Publication of the grant of the patent:
**02.04.86 Bulletin 86/14**

(84) Designated Contracting States:
**AT BE CH DE FR GB LI NL**

(56) References cited:
**FR-A-2 416 039**
**US-A-4 160 801**
**US-A-4 183 961**

(73) Proprietor: **DIDECO S.p.A.**
**Via Galvani, 6**
**I-41037 Mirandola (Province of Modena) (IT)**

(72) Inventor: **Luppi, Libero**
**Viale Italia, 62**
**I-41037 Mirandola (province of Modena) (IT)**

(74) Representative: **Modiano, Guido et al**
**MODIANO & ASSOCIATI S.A.S. Via Meravigli, 16**
**I-20123 Milan (IT)**

Courier Press, Leamington Spa, England.

## Description

This invention relates to a device for oxygenating blood in extrabodily circulation with heat exchanger.

It is known that in the course of some surgical interventions it becomes necessary to circulate blood externally to the patient's body, during which extrabodily circulation blood requires to be oxygenated and heated in order to maintain the normal body temperature, or cooled where momentarily operating in hypothermal conditions and, for this purpose devices have been proposed, such as "method of oxygenating blood", as disclosed in prior U.S. Patent No. 1,183,961 to Curtis known as oxygenators, some of which include a heat exchanger capable of performing the above functions but are not devoid of shortcomings.

It will be appreciated, first of all, that a basic requirement of an oxygenating device is that blood must flow through it in optimum conditions, both from the standpoint of enabling a good intermixing of oxygen, and from that of preventing stagnation points or the throwing of blood against the walls, which could have fatal results through hemolysis phenomena, i.e. the collapsing of red cells, and from the very important standpoint of minimizing the contact of blood with the walls, which constitute a foreign object susceptible to become a source of contamination and other complications.

The optimization of the flow is of course achieved with a suitable selection of the cross-section dimensions of the passageways within the oxygenator in accordance with the blood flow rate; this flow rate varying in a directly proportional way to the patient's weight, conventional devices are manufactured in three different sizes, one of which is called "infant" and intended for newly born children, one for pediatric applications, and one for adult applications, in an attempt at approaching as far as possible the optimum condition in each case; however it is evident that the optimization thus achieved can only be a coarse one because each of the three types will have to accommodate widely different flow rates both on account of the different weights of the patients being treated and because any individual patient may involve momentarily different conditions especially when the intervention is carried out in conditions of hypothermy, with attendant greatly reduced blood flow rate.

Moreover, the availability of three sizes of the oxygenator leads to known problems of supplying and inventory, such as arise whenever an article happens to be an assorted one. To the coarse optimization of the sizes of the oxygenating devices as a function of the blood flow rate is then connected another negative aspect of conventional devices consisting in that the priming, i.e. the amount of blood which must be retained within the apparatus in order for its operation to be primed, does not follow the flow rate drops proportionally, disproportionately high values constantly being maintained, thus subtracting blood from the amount required by the surgeon to face contingent situations.

Thus, the task of the invention is to eliminate such prior drawbacks by providing an oxygenating device which can accommodate different blood flow rates in an optimum manner, as required by the varying weights of the patients, from newly born children to adults.

Within said task it is an object of the invention to provide an oxygenating device wherein the heat exchanger presents a large exchange surface area for any blood flow rate, thus affording short times particularly for the heating step which follows a hypothermal stage.

Another object of the invention is to provide an oxygenating device of small size and cost.

According to one aspect of the present invention the aforesaid task and objects are achieved by a device for oxygenating blood in extrabodily circulation with heat exchanger, a plurality of selectively operable oxygenating modules, each module comprising at least one diffuser of oxygen (9, 10) through the blood, each diffuser having a closure member (17, 18) for simultaneously shutting off the flow of the two fluids, the diffusers being included in a distributor into which a venous blood manifold (3) and an oxygen manifold (5) open, and a chamber (22) underlying the diffusers, the pipes (24) of the heat exchanger extending through the chamber (22), said oxygenating modules being in communication, via a layer of an antifoaming material (35, 36), with an arterial blood collecting and storing chamber (38) which is provided with outlet fittings (39, 40).

With a device of this type, it will be apparent how, by suitably manipulating the shutter members, it becomes possible to operate the most suitable number of oxygenation modules in accordance with the required flow rate, thus achieving a much sophisticated optimization of the blood flow conditions; consequently, optimum oxygenation, a continuous stagnation-free and splash-free flow, and minimal blood-to-walls contact are achieved as well as a priming action which is strictly a function of the flow rate.

Further features and advantages will become more clearly apparent from the following description of a preferred but not limitative embodiment of the invention, as illustrated by way of example only in the accompanying drawings, where:

Figure 1 is a perspective view of the device according to the invention showing some members partly cut away and in ghost lines;

Figure 2 shows a section, interrupted at the middle area, of said device with the cross plane including the axis of the first diffuser shown in the cut away view of Figure 1;

Figure 3 is an isometric view, with members shown in ghost lines, of a withdrawal tap; and

Figure 4 is an exploded view of the venous blood inlet fitting.

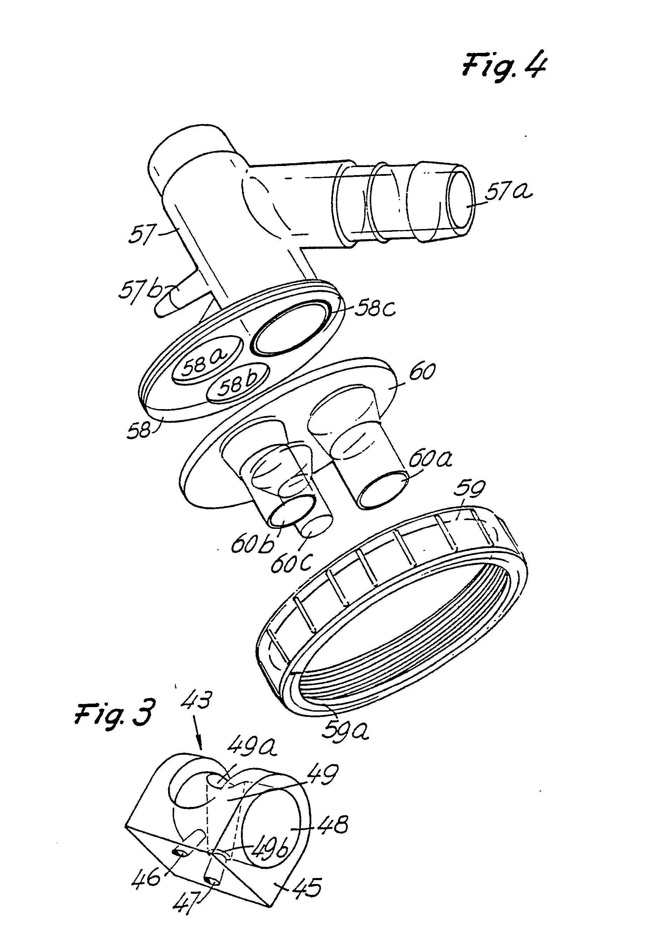

With reference to the drawing figures, there is indicated at 1 the distributor to which is delivered venous blood which enters the device through the connector or fitting 2, which will be described in detail hereinafter with reference to Figure 4, the venous blood then flows through the manifold 3, oxygen, introduced through the fitting 4, also flows through the manifold 5, said manifold 5 being formed within the cover 6 which comprises the filter 7 bearing against the supporting plates 8.

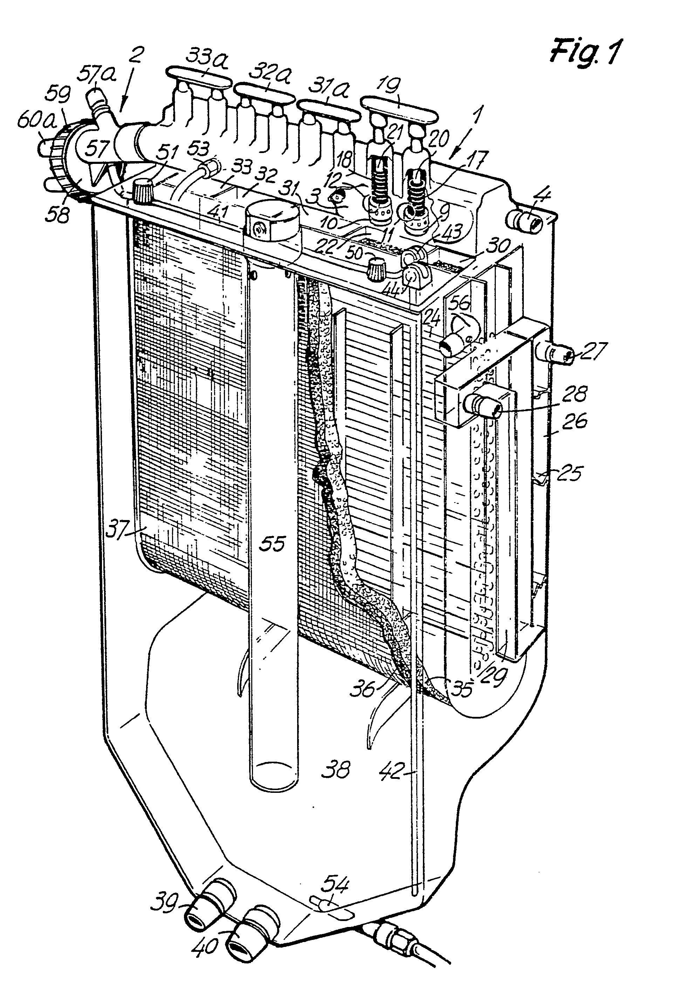

The oxygenation module, shown cut away in Figure 1, comprises the two oxygen diffusers made up of the stub pipes 9 and 10 communicating, through the holes 11 and 12, with the venous blood manifold 3 and being formed through the walls with holes as at 13 for the diffuser 9 through which oxygen is admitted to mix with blood from wells, as at 14, communicating through holes, as at 15, with the chamber 16 whereinto the oxygen is introduced through the filter 7.

The two stub pipes 9 and 10 are equipped with blood and oxygen closure members comprising plungers 17 and 18 made of an elastic material, such as rubber, and being shown in the figures at their stroke bottom end in position to shut off both fluids.

The concurrent sliding movement of the two plungers in contact with the walls of the related diffuser and those of a hole such as 9a for the diffuser 9 to reach their top stroke end into a position of free admission of oxygen and blood, that is with their downward faces at a higher level than the hole 11, is accomplished by pulling up the handle 19 which connects the small rods 20 and 21 rigid with the plungers 17 and 18.

The oxygenation module being described, which comprises the diffusers 9 and 10, also comprises the underlying chamber 22 whereinto the blood is introduced by gravity from said diffusers to first meet a non-siliconed layer of polyurethane 23 which deadens the impact and homogenizes the blood and oxygen foam to facilitate the mixing, and subsequently the pipes, as at 24, of a heat exchanger adapted for conveying a flow of water, suitably directed by partitions, as at 25, which reaches the manifold 29 through the fitting 28 and leaves through the fitting 29 of the manifold 26 located at the same level as the fitting 28 to prevent the exchanger from becoming emptied; advantageously, the pipes, as at 24, are arranged in staggered rows along perpendicular directions to the flow of blood sweeping them externally, that is horizontal in the normal operating position of the device, thereby a sort of blood lamination is produced with attendant increase of the contact area thereof with the individual oxygen bubbles.

In addition to the oxygenation module just described, the device, as illustrated in Figure 1, also includes the module comprising the chamber 30 and overlying diffusers, not shown, the blood and oxygen being introduced into said chamber at all times during the operation because the diffusers are not equipped with closure members, and the modules formed by the chambers 31, 32 and 33 with their related overlying diffusers, not shown, with the shut-off members are operable by manipulating the handles 31a, 32a and 33a, respectively.

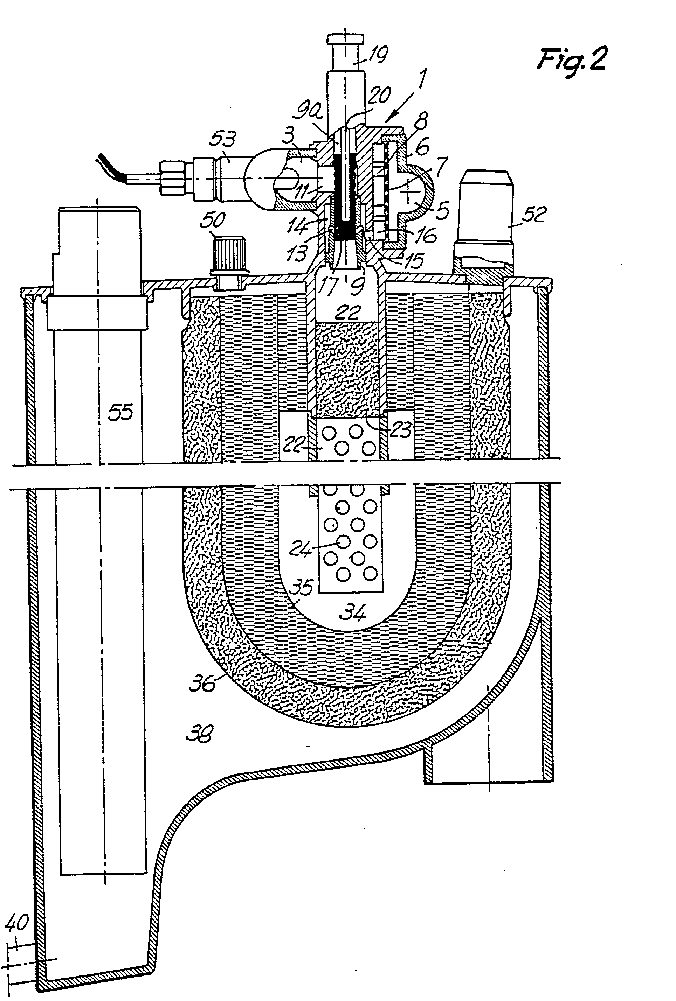

The foaming blood which leaves the chambers of the modules described hereinabove reaches the space indicated at 34 in Figure 2, and hence, through the layers 35 and 36 of a suitable two-grade siliconed material with antifoaming functions and through the filter screen 37, reaches the collecting and storing chamber 38 equipped with outlet fittings 39 and 40.

There are indicated at 41 and 42 two small pipes respectively connected to the venous blood inlet fitting 2 and arterial blood collecting chamber 38, connected to the taps 43 and 44 for the spilling of samples; said taps are identical, and the one indicated at 43 will be described in detail with reference to Figure 3.

Said tap comprises the fixed body 45 formed with the passage 46 connected to the small pipe 41 and with the passage 47 connected to the collecting chamber 38, and the rotatable body 48 having the bore 49 which can be brought, by manual operation, onto a syringe inserted into the end 49a, with the end 49b at one of the two passages 46 and 47.

The withdrawal is effected by first extracting with the syringe the blood contained in the small pipe 41, considered non-valid for sampling, through the passage 46, and then ejecting said blood, after a suitable rotation of 48, through the passage 47, and thereafter again extracting through 46 the blood renewed in 41, after the return rotation of 48. Indicated at 50 and 51 are then two fittings for the introduction of substances, at 52 a fitting for quick filling, at 53 and 54 two probes for detecting the temperature, at 55 a probe for detecting the level of the blood in the chamber 38, and at 56 an outlet fitting for the gases to be removed from the device interior with an anti-implosion hole.

Finally, and with reference to Figure 4, the venous blood inlet connector 2 will be described. Said connector includes the fitting 57 connected with one end to the manifold 3 and having at its other end the flange 58, formed with seats 58a and 58b and having the gasket 58c at the hole for the fitting 57, which is threaded on the outer edge to match the inside threads of the ring nut 59 which has an abutment 59a for retention and compression against the flange 58 of the disk 60; said disk has three holes at the three stub pipes 60a, 60b and 60c of different diameters, the most suitable one for infant use, or pediatric, or adult applications, being positioned at the hole of the fitting 57. On said fitting 57, there are provided a branching off 57a for connection to a cardiotome and a branch-off 57b for connection to the small pipe 41 for the withdrawal of venous blood samples.

It will be appreciated from the foregoing description how, with extremely simple manipulations, it becomes possible to determine at any time the optimum cross-section for the blood passage by admitting the same to the most suitable number of oxygenation modules, thus achieving all of the advantages described hereinabove, that is optimum admixture of oxygen, a splash-free flow with minimal contact with the wall; moreover the reduction of priming to strictly indispensable amounts for each flow rate can cause the involvement of a minimal amount of antifoaming material.

Also noteworthy is the feature of the oxygen and venous blood closure member to each oxygenation module, which closure member is effective to stop the flow of the two fluids in an almost simultaneous way, thus avoiding the problems which may result from the stopping of the flow of just one of said fluids.

Another important aspect of the invention resides in that the heat exchanger is provided with a large width dimension, and ensures, even with the smallest flow rates, a large exchange surface, contrary to what happens in the known devices where the exchangers for the infant or pediatric sizes will be of reduced dimensions.

The invention described above is susceptible to many modifications and variations without departing from the scope of the instant inventive concept; it is evident, for example, that the number of the oxygenation modules may be different from the one shown, that is five, and that for each module the number of the diffusers may be different from the two described.

Furthermore, nothing changes in principle where the blood inlet is located at any other points with consequent variation of the flow direction.

In practicing the invention, all of the details may be replaced with other technically equivalent elements; the materials used, as well as the shapes and dimensions, may be any suitable ones for the intended application.

**Claims**

1. A device for oxygenating blood in extrabodily circulation with heat exchanger, a plurality of selectively operable oxygenating modules, each module comprising at least one diffuser of oxygen through the blood, each diffuser having a closure member for simultaneously shutting off the flow of the two fluids, the diffusers being included in a distributor into which a venous blood manifold and an oxygen manifold open, and a chamber underlying the diffusers, pipes of the heat exchanger extending through the chamber, said oxygenating modules being in communication, via a layer of an antifoaming material, with an arterial blood collecting and storing chamber which is provided with outlet fittings.

2. A device according to Claim 1, characterized in that the diffuser of oxygen through the blood comprises a stub pipe (9, 10) arranged with its axis substantially parallel to the longitudinal axis of the chamber (22), the stub pipe being in communication with the venous blood manifold (3), and with the oxygen manifold (5) via inclined holes (13) in the wall of the pipe.

3. A device according to Claim 2, characterized in that the oxygen manifold (5) leading from the distributor is formed within a cover (6) of said distributor and is adapted to clamp in position a membrane constituting an oxygen filter (7).

4. A device according to Claim 1, characterized in that the closure member (17, 18) belonging to each diffuser of oxygen through the blood comprises a plunger (17, 18) of an elastic material such as rubber, the plunger being slidable and in contact with the inner wall of the diffuser and with the wall of a bore (9a) formed coaxial therewith in the body of the distributor between the two limit positions of complete shut-off of flow of both fluids and unhindered admission of the same to the chamber, the plungers (17, 18) of the diffusers belonging to one module being provided with interconnected operating members (20, 21).

5. A device according to Claim 4, characterized in that the operating member (20, 21) belonging to each plunger (17, 18) comprises a metal rod (20, 21) extending from the opposite face of the plunger to that facing the chamber (22), and protruding out of the distributor to connect with a handle (19) which in turn connects the ends of the rods (20, 21) extending from the plungers (17, 18) belonging to the same oxygenation module.

6. A device according to one or more of the preceding claims, characterized in that a layer of non-siliconed polyurethane (23) is provided in the upper region of the chamber (22).

7. A device according to one or more of the preceding claims, characterized in that the pipes (24) of the heat exchanger are adapted for conveying the water which enters and leaves through connectors (28, 29) located at the same level, and is intended for supplying or extracting heat to/from the blood, the pipes arranged in staggered rows perpendicularly to the direction of the blood flow in a space delimited by walls extending substantially vertically in the normal position of use of the device and opening to a space delimited by said antifoaming material (35, 36).

8. A device according to one or more of the preceding claims, characterized in that said antifoaming material (35, 36) interposed between the oxygenation modules and the arterial blood collecting chamber (38) comprises a double layer (35, 36) of a material suitably siliconed to different grades, delimited on the side of the collecting chamber (38) by a filtering mesh (37).

9. A device according to one or more of the preceding claims, characterized in that the venous blood inlet connector (2) in the related manifold (3) comprises a fitting (57) connected with one end to said manifold (3) and being provided, at the other end thereof, with a flange (58) having a threaded edge adapted for engaging the inside threads of a ring nut (59) having an abutment (59a) for retention and compression against said flange (58) of a disk member (60) drilled for the connection of stub pipes (60a, 60b, 60c) of varying diameters.

10. A device according to Claim 9, characterized in that the fitting (57) has a first branch-off (57a) for connection to a cardiotome, and a second branch-off (57b) for connection to a small sampling pipe (41).

11. A device according to Claim 9, characterized in that the stub pipes of varying diameters are three in number, correspondingly to the optimization of the flow for the three applications of the device, in the infant, pediatric and adult modes thereof.

12. A device according to one or more of the preceding claims, characterized in that it comprises two small pipes (41, 42) with one end at the venous blood inlet (2) and arterial blood outlet (38), respectively, and the other end connected to a withdrawal tap (43, 44).

13. A device according to Claim 12, characterized in that each withdrawal tap (43, 44) comprises a fixed body (45) having a passage (46) connected to a said small pipe (41) and a passage (47) connected to the arterial blood collecting chamber (38), and a rotary body (48) having a substantially diametrical bore (49) which can be brought by manual actuation with one end (49b) at one or the other of the passages (46, 47) through the fixed body (45), the other end (49a) being adapted for connection to a syringe.

14. A device according to one or more of the preceding claims, characterized in that it comprises an outlet connector (56) for the gases released from the interior, and a safety anti-implosion valve effective to protect against implosions as said connector (56) is connected to a forced vacuum line.

15. A device according to one or more of the preceding claims, characterized in that it comprises an arterial blood level probe (55) in the collecting chamber (38).

16. A device according to one or more of the preceding claims, characterized in that it comprises a probe (53, 54) for detecting the blood temperature at the venous blood inlet and arterial blood outlet.

17. A device according to one or more of the preceding claims, characterized in that it comprises fittings (50, 51) for introducing substances into the blood and for quick filling.

**Patentansprüche**

1. Vorrichtung zur Sauerstoffanreicherung von Blut in einem Kreislauf ausserhalb des Körpers mit Wärmetauscher, einer Vielzahl von selektiv in Betrieb setzbare Sauerstoffanreicherungsmodulen, von denen jeder mindestens eine Diffusionsvorrichtung zur Hindurchdiffundierung von Sauerstoff durch das Blut, wobei jede Diffundiervorrichtung ein Verschlussteil zur gleichzeitigen Sperrung des Stromes der beiden Fluiden besitzt, wobei die Diffundiervorrichtungen in einem Verteiler eingeschlossen sind, in den ein Sammelkopf für venöses Blut und ein Sammelkopf für den Sauerstoff münden, und einer Kammer unterhalb der Diffusionsvorrichtungen, durch welche Kammer sich Wärmetauscherrohre erstrecken, wobei die Sauerstoffanreicherungsmodule über eine Schicht eines Antischäummaterials mit einer Sammel- und Speicherkammer für arterielles Blut in Verbindung stehen, die mit Auslassanschlüssen versehen ist.

2. Vorrichtung nach Anspruch 1, dadurch gekennzeichnet, dass die Diffusionsvorrichtung für den Sauerstoff durch das Blut einen Rohrstutzen (9, 10) aufweist, der mit seiner Achse im wesentlichen parallel zur Längsachse der Kammer (22) angeordnet ist und mit dem Sammelkopf für venöses Blut (3) und mit dem Sammelkopf für den Sauerstoff (5) über Schräglöcher (13) in der Wand des Rohrstutzens in Verbindung steht.

3. Vorrichtung nach Anspruch 2, dadurch gekennzeichnet, dass der vom Verteiler wegführende Sammelkopf für den Sauerstoff (5) in einem Deckel (6) des Verteilers ausgebildet ist und befähigt ist, eine einen Sauerstoffilter (7) bildende Membrane in ihrer Lage eingeklemmt zu halten.

4. Vorrichtung nach Anspruch 1, dadurch gekennzeichnet, dass das einer jeden Diffundiervorrichtung für den Sauerstoff durch das Blut angehörende Verschlussteil (17, 18) aus einem Kolben (17, 18) aus elastischem Material, wie Gummi, besteht, der verschiebbar und in Berührung mit der Innenwand der Diffundiervorrichtung und mit der Wand einer Öffnung (9a) angeordnet ist, die koaxial zum Kolben im Körper des Verteilers zwischen den beiden Endstellungen der vollständigen Sperrung des Stromes der beiden Fluide und des ungehinderten Zuflusses derselben zur Kammer ausgebildet ist, wobei die Kolben (17, 18) der einem Modul angehörenden Diffundiervorrichtungen mit untereinander verbundenen Stellgliedern (20, 21) versehen sind.

5. Vorrichtung nach Anspruch 4, dadurch gekennzeichnet, dass die einem jeden Kolben (17, 18) angehörenden Stellglieder (20, 21) aus einem Metallstab (20, 21) bestehen, der sich von der Seite des Kolbens erstreckt, die zur Kammer (22) zugewandten Seite entgegengesetzt ist, und aus dem Verteiler herausragt und mit einem Handgriff (19) verbunden ist, der seinerseits die Enden der Stäbe (20, 21) verbindet, die sich von den zum selben Sauerstoffanreicherungsmodul gehörenden Kolben (17, 18) erstrecken.

6. Vorrichtung nach einem oder mehreren der vorhergehenden Ansprüche, dadurch gekennzeichnet, dass im oberen Bereich der Kammer (22) eine Schicht aus nicht siliconisiertem Polyurethan (23) vorgesehen ist.

7. Vorrichtung nach einem oder mehreren der vorhergehenden Ansprüche, dadurch gekennzeichnet, dass die Rohre (24) des Wärmetauschers das Wasser führen, welches durch in derselben Höhe liegende Anschlüsse (28, 29) eintritt und austritt und Wärme dem Blut zuführt oder diesem entnimmt, wobei die Rohre in gestaffelten Reihen senkrecht zur Richtung des Blutflusses in einem Raum angeordnet sind, der durch Wände begrenzt ist, die sich senkrecht bei normaler Benützungsstellung der Vorrichtung erstrecken und in einem durch das Antischäummaterial (35, 36) begrenzten Raum enden.

8. Vorrichtung nach einem oder mehreren der vorhergehenden Ansprüche, dadurch gekennzeichnet, dass das zwischen den Sauerstoffanreicherungsmodulen und der Sammelkammer für das Arterienblut (38) eingesetzte Antischäummaterial (35, 36) aus einer Doppelschicht (35, 36) eines in entsprechender Weise in verschiedenem Ausmass silikonisierten Materials besteht, das an der Seite der Sammelkammer (38) durch ein Filtergeflecht (37) begrenzt ist.

9. Vorrichtung nach einem oder mehreren der vorhergehenden Ansprüche, dadurch gekennzeichnet, dass der Einlassanschluss für das venöse Blut (2) im betreffenden Sammelkopf (3) aus einem Anschlussstück (57) besteht, das mit einem Ende am Sammelkopf (3) angeschlossen und am anderen Ende mit einem Flansch (58) mit Gewinderand versehen ist, der in das Innengewinde einer Ringmutter (59) einschraubbar ist, die einen Anschlag (59a) aufweist, um einen Scheibenteil (60) zu halten und gegen den Flansch (58) zu pressen, welcher Scheibenteil für den Anschluss von Rohrstützen (60a, 60b, 60c) verschiedenen Durchmessers gelöchert ist.

10. Vorrichtung nach Anspruch 9, dadurch gekennzeichnet, dass das Anschlussstück (57) eine erste Abzweigung (57a) für die Verbindung mit einem Cardiotom und eine zweite Abzweigung (57b) für die Verbindung mit einem kleinen Probeentnahmerohr (41) aufweist.

11. Vorrichtung nach Anspruch 9, dadurch gekennzeichnet, dass die Rohrstutzen verschiedenen Durchmessers in einer Anzahl von drei in Übereinstimmung mit der Optimierung des Flusses für die drei Anwendungsarten der Vorrichtung im Kindermodus, Jugendlichenmodus und Erwachsenenmodus vorgesehen sind.

12. Vorrichtung nach einem oder mehreren der vorhergehenden Ansprüche, dadurch gekennzeichnet, dass sie zwei kleine Rohre (41, 42) aufweist, deren ein Ende im Einlass für das venöse Blut (2) bzw. dem Auslass für das arterielle Blut (38) liegt und deren anderes Ende an einen Entnahmehahn (43, 44) angeschlossen ist.

13. Vorrichtung nach Anspruch 12, dadurch gekennzeichnet, dass jeder Entnahmehahn (43, 44) einen fixen Körper (45) mit einem an das Rohr (41) angeschlossenen Durchlass (46), und einem an die Sammelkammer für das arterielle Blut (38) angeschlossenen Durchlass (47) und einen Drehkörper (48) mit einer im wesentlichen diametralen Bohrung (49) aufweist, welche durch manuelle Betätigung mit einem Ende (49b) zu dem einen oder dem anderen der Durchlässe (46, 47) im fixen Körper (45) gebracht werden kann, wogegen das andere Ende (49a) an eine Spritze anschliessbar ist.

14. Vorrichtung nach einem oder mehreren der vorhergehenden Ansprüche, dadurch gekennzeichnet, dass es einen Auslassanschluss (56) für die im Inneren freigesetzten Gase und ein Sicherheits-Antiimplosionsventil, welches wirksam gegen Implosionen bei Anschluss des Auslassanschlusses (56) an eine Vakuumleitung schützt, aufweist.

15. Vorrichtung nach einem oder mehreren der vorhergehenden Ansprüche, dadurch gekennzeichnet, dass es in der Sammelkammer (38) einen Fühler (55) für den arteriellen Blutspiegel aufweist.

16. Vorrichtung nach einem oder mehreren der vorhergehenden Ansprüche, dadurch gekennzeichnet, dass es einen Fühler (53, 54) für die Ermittlung der Bluttemperatur an Einlass für das venöse Blut und am Auslass für das arterielle Blut aufweist.

17. Vorrichtung nach einem oder mehreren der vorhergehenden Ansprüche, dadurch gekennzeichnet, dass sie Anschlüsse (50, 51) für die Einführung von Substanzen in das Blut und zur Schnellfüllung aufweist.

**Revendications**

1. Dispositif pour l'oxygénation du sang en circulation extracorporelle, comprenant un échangeur de chaleur, une pluralité de modules d'oxygénation à fonctionnement sélectif, chaque module comprenant au moins un diffuseur d'oxygène dans le sang, chaque diffuseur comportant un organe d'obturation pour interrompre simultanément le débit des deux fluides, les diffuseurs étant inclus dans un distributeur dans lequel débouche un collecteur de sang veineux et un collecteur d'oxygène, et une chambre située sous les diffuseurs, des tubes de l'échangeur de chaleur s'étendant à travers la chambre, les modules d'oxygénation étant en communication, par l'intermédiaire d'une couche d'une matière anti-moussage, avec une chambre de collecte et de stockage de sang artétiel qui est munie de raccords de sortie.

2. Dispositif suivant la revendication 1, caractérisé en ce que le diffuseur d'oxygène dans le sang comprend un tube (9, 10) dont l'axe est sensiblement parallèle à l'axe longitudinal de la chambre (22), ce tube étant en communication avec le collecteur (3) de sang veineux, et avec le collecteur (5) d'oxygène par l'intermédiaire de trous inclinés (13) prévus dans la paroi du tube.

3. Dispositif suivant la revendication 2, caractérisé en ce que le collecteur d'oxygène (5) aboutissant au distributeur est défini à l'intérieur d'un couvercle (6) de ce distributeur et est prévu pour bloquer en position une membrane constituant un filtre à oxygène (7).

4. Dispositif suivant la revendication 1, caractérisé en ce que l'organe d'obturation (17, 18) appartenant à chaque diffuseur d'oxygène dans le sang comprend un plongeur (17, 18) en matière élastique telle que le caoutchouc, le plongeur pouvant coulisser et étant en contact avec la paroi intérieure du diffuseur et avec la paroi d'un alésage (9a) coaxial au plongeur dans le corps du distributeur, entre les deux positions extrêmes d'obturation complète du débit des deux fluides et d'admission libre de ces derniers dans la chambre, les plongeurs (17, 18) des diffuseurs appartenant à un même module comportant des éléments de manoeuvre interconnectés (20, 21).

5. Dispositif suivant la revendication 4, caractérisé en ce que l'élément de manoeuvre (20, 21) appartenant à chaque plongeur (17, 18) comprend une tige de métal (20, 21) qui s'étend de la face opposée du plongeur à celle en regard de la chambre (22), et qui fait saillie à l'extérieur du distributeur de manière à être reliée à une poignée (19) qui relie elle-même les extrémités des tiges (20, 21) venant des plongeurs (17, 18) qui appartiennent au même module d'oxygénation.

6. Dispositif suivant une ou plusieurs des revendications précédentes, caractérisé en ce qu'une couche de polyuréthane non siliconé (23) est prévue dans la région supérieure de la chambre (22).

7. Dispositif suivant une ou plusieurs des revendications précédentes, caractérisé en ce que les tubes (24) de l'échangeur de chaleur sont prévus pour la circulation de l'eau qui entre et sort par des raccords (28, 29) situés au même niveau et qui est destinée à fournir de la chaleur au sang ou à en extraire de celui-ci, les tubes étant disposés en rangées décalées, perpendiculairement à la direction d'écoulement du sang dans un espace délimité par des parois qui s'étendent sensiblement verticalement dans la position normale d'utilisation du dispositif et débouchant dans un espace délimité par ladite matière anti-moussage (35, 36).

8. Dispositif suivant une ou plusieurs des revendications précédentes, caractérisé en ce que la matière anti-moussage (35, 36), interposée entre les modules d'oxygénation et la chambre (38) de collecte de sang artériel, comprend une double couche (35, 36) d'une matière convenablement siliconée à des degrés différents, délimitée sur le côté de la chambre de collecte (38) par un tamis filtrant (37).

9. Dispositif suivant une ou plusieurs des revendications précédentes, caractérisé en ce que le connecteur (2) d'entrée de sang veineux dans le collecteur correspondant (3) comprend un raccord (57) relié à une extrémité à ce collecteur (3) et comportant, à son autre extrémité, une bride (58) à bord fileté prévu pour se visser dans le filetage intérieur d'un écrou annulaire (59) présentant un épaulement (59a) de retenue et de compression, contre la bride (58), d'un disque (60) percé pour le raccordement de tubes (60a, 60b, 60c) de différents diamètres.

10. Dispositif suivant la revendication 9, caractérisé en ce que le raccord (57) comporte un premier branchement (57a), pour la liaison à un cardiotome, et un deuxième branchement (57b) pour la liaison à une petite tuyauterie d'échantillonnage (41).

11. Dispositif suivant la revendication 9, caractérisé en ce que les tubes de différents diamètres sont au nombre de trois, afin de correspondre à l'optimisation du débit pour les trois applications du dispositif, dans ses modes pour enfant, pédiatrique et pour adulte.

12. Dispositif suivant une ou plusieurs des revendications précédentes, caractérisé en ce qu'il comprend deux petites tuyauteries (41, 42), dont une extrémité est reliée à l'entrée (2) de sang veineux et à la sortie (38) de sang artériel, respectivement, et dont l'autre extrémité est reliée à un robinet de prélèvement (43, 44).

13. Dispositif suivant la revendication 12, caractérisé en ce que chaque robinet de prélèvement (43, 44) comprend un corps fixe (45), comportant un passage (46) raccordé à une dite petite tuyauterie (41) et un passage (47) raccordé à la chambre (38) de collecte de sang artériel, et un élément tournant (48) comportant un orifice sensiblement diamétral (49) qui peut être amené, par manoeuvre manuelle avec une extrémité (49b) à l'un ou à l'autre des passages (46, 47) à travers le corps fixe (45), l'autre extrémité (49a) étant prévue pour la liaison à une seringue.

14. Dispositif suivant une ou plusieurs des revendications précédentes caractérisé en ce qu'il comprend un connecteur de sortie (56) pour les gaz évacués de l'intérieur, et une soupape de sécurité anti-implosion qui sert à la protection contre les implosions lorsque ledit connecteur (56) est raccordé à une conduite de vide forcé.

15. Dispositif suivant une ou plusieurs des revendications précédentes, caractérisé en ce qu'il comprend une sonde (55) de niveau de sang artériel dans la chambre de collecte (38).

16. Dispositif suivant une ou plusieurs des revendications précédentes, caractérisé en ce qu'il comprend une sonde (53, 54) de détection de la température du sang à l'entrée de sang veineux et à la sortie de sang artériel.

17. Dispositif suivant une ou plusieurs des revendications précédentes, caractérisé en ce qu'il comprend des raccords (50, 51) pour l'introduction de substances dans le sang et pour le remplissage rapide.

Fig.1

*Fig.2*

0 049 461

*Fig.4*

*Fig.3*

3